# EUROPEAN PATENT SPECIFICATION

(11) **EP 1 542 804 B1**
(45) Date of publication and mention of the grant of the patent: **08.06.2011**
(21) Application number: 03754759.3
(22) Date of filing: 19.09.2003
(51) Int. Cl.: B01L 3/14

(54) **HIGH BIAS GEL TUBE AND PROCESS FOR MAKING TUBE**
VERSUCHSBEHÄLTER MIT SCHRÄGEM GEL UND VERFAHREN ZUR HERSTELLUNG
TUBE DE GEL HAUTEMENT SOLLICITE ET PROCEDE DE FABRICATION DU TUBE

(30) Priority: 23.09.2002 US 412824 P
(43) Date of publication of application: 22.06.2005
(60) Divisional application: 10178801.6
(73) Proprietor: Becton Dickinson and Company, Franklin Lakes, New Jersey 07417-1880 (US)
(72) Inventor: MANOUSSAKIS, Dimirios, Wyckoff, NJ 07481 (US); BRADSHAW, Allen, Sumter, SC 29153 (US); MARTIN, Paul, Plymouth PL17PW (GB)
(74) Representative: von Kreisler Selting Werner
(86) International application number: PCT/US2003/029508
(87) International publication number: WO 2004/026477

(56) References cited:
- FR-A- 2 640 753
- US-A- 3 852 194
- US-A- 3 997 442
- US-A- 4 426 290
- US-B1- 6 238 578

## Description

### Field of the Invention

The invention relates to body fluid collection containers, in particular blood collection tubes, capable of separating phases of different density, using a gel separating medium.

### Discussion of the Related Art

Fluid collection tubes containing a thixotropic gel for separating phases of different densities, e.g., in blood, are well known. See, e.g., U.S. -A-3,997,442, 4,257,886, 4,426,290, 4,770,779, and 6,238,578.
The gel is selected to have a density between that of the phases of blood which are to be separated. Upon centrifugation of a collected blood sample, the force of centrifugation forces the gel from a substantially non-flowing state to a more flowable state. In the flowable state, the gel migrates to a position between the two phases, e.g., between serum and clot portions. And upon cessation of centrifugation, the gel again becomes substantially non-flowable, thereby maintaining the separation between phases. Gel movement, i.e., getting adequate movement of the gel upon centrifugation, can sometimes be an issue. U.S. -A-3,997,442 suggests one solution, but improvements are always desired.

### SUMMARY OF THE INVENTION

The invention relates to an improved fluid collection container, containing a gel separation medium. According to the invention, the gel is disposed in the tube in a manner and geometry that is readily manufacturable, and which overcomes potential gel movement issues.
In particular, the present invention relates to a container comprising an upper end, a lower end, and a sidewall between the upper and lower ends having inner and outer walls; and a gel located in the container in contact with a portion of the inner wall, wherein the gel comprises continuous first and second regions, the first region located at or adjacent to the lower end, and the second region extending upward from a portion of the first region, characterized in that the first region comprises an imaginary upper boundary at which the first region exhibits 360° circumferential contact with the inner wall, wherein the first region comprises at least 80 vol.% of the gel and wherein the entirety of the second region exhibits less than 180° circumferential contact with the inner wall.
Furthermore, the present invention relates to a process for fabricating said container, said process comprising the steps of: providing a container having an open upper end, a lower end, and a sidewall between the upper and lower ends having inner and outer walls; disposing into the container a gel at an angle to the longitudinal axis of the container; and allowing the gel to slump.
Preferred embodiments become apparent from the dependent claims.

### BRIEF DESCRIPTION OF THE DRAWINGS

Fig. 1 shows a tube containing a separator gel material according to an aspect of the invention.

Fig. 2 shows a cross-sectional view of a tube containing a separator gel material according to an aspect of the invention.

Fig. 3 shows a cross-sectional view of a tube containing a separator gel material according to an aspect of the invention.

Fig. 4 shows a cross-sectional view of a tube containing a separator gel material according to an aspect of the invention.

Fig. 5 shows a cross-sectional view of a tube containing a separator gel material according to an aspect of the invention.

Figs. 6A-6C show cross-section profiles for a tube containing a separator gel material according to an aspect of the invention.

Figs. 7A-7G show cross-sectional profiles for a tube containing a separator gel material according to an aspect of the invention.

Fig. 8 shows a cross-sectional view of a tube containing a separator gel material according to an aspect of the invention.

### DETAILED DESCRIPTION OF THE INVENTION

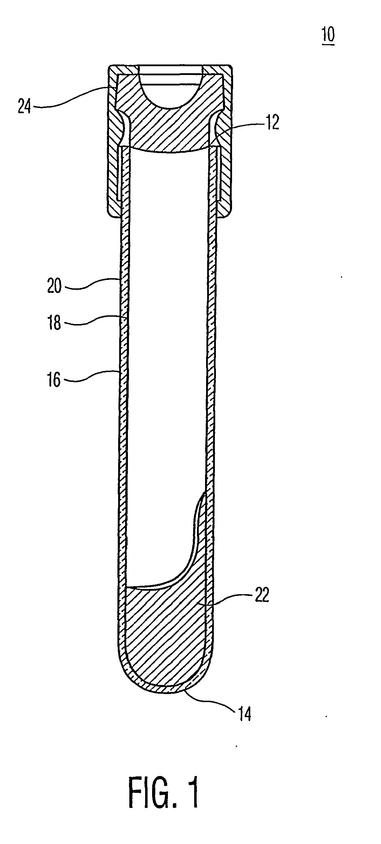

A typical blood collection tube according to the invention is shown in Fig. 1. The tube 10 contains an open upper end 12, a lower closed end 14, and sidewalls 16 having an inner wall 18 and an outer wall 20. A separating gel 22 is located within the container, at or adjacent the closed end 14.

The tube 10 is provided with a pierceable cap 24, that may be pierced by the non-patient end of a double ended blood collection needle. The tube 10 is generally evacuated, such that upon piercing by such a needle, blood is drawn into the tube. Details of evacuated blood collection tubes and blood collection are well known to those skilled in the art.

As noted above, upon sample collection, the tube is centrifuged to separate two phases of the blood sample, e.g., serum and red blood cells, or different cell types, as known in the art

The invention provides the gel in the tube in an advantageous manner, that avoids or overcomes issues relating to gel movement

According to the invention, a tube is provided with a gel separating material having an initial state that reflects an intermediate, transient state (during centrifugation) of a typical gel. In particular, the gel exhibits a state prior to any centrifugation that substantially resembles an intermediate state of an identical gel undergoing centrifugation in an identical container, wherein the initial state of the identical gel comprises an identical volume of the gel exhibiting a substantially planar exposed top surface. For example, where the exposed top surface of the identical gel exhibits a best-fit plane that exhibits an angle of 0 to 20° to a plane perpendicular to the longitudinal axis of the tube, the initial gel configuration of the invention would reflect an intermediate (during centrifugation) state of that identical gel.

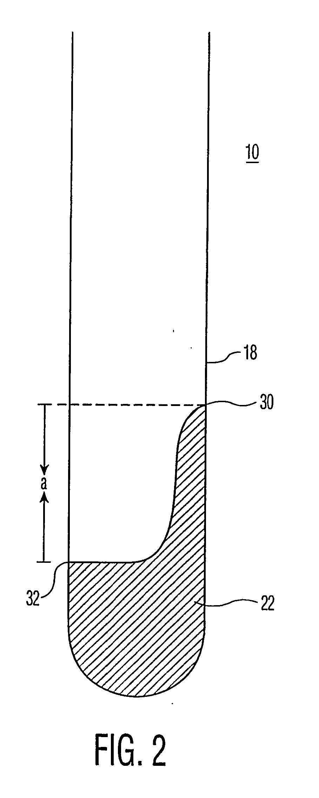

Embodiments of the gel location/geometry, from the outside of the tube, as well as some cross-section views, are shown in Figs. 2 to 9. It is possible to obtain the advantages of the invention by disposing the gel into the tube using a variety of principles and guidelines. The Figures show one type of design only, which is representative of the design guidelines presented herein. Variations based on the principles and description herein are also contemplated.

In one embodiment, reflected in Fig. 2, the distance a between the uppermost point 30 at which the gel 22 contacts the inner wall 18, and the highest point 32 at which the gel contacts the inner wall roughly opposite to the uppermost point, i.e., from 90° to 270° circumferentially, typically from 120° to 240°, most often including at least 180° circumferentially, is at least 8 mm, typically 8 to 21 mm. Typically in this embodiment the gel, along a plane perpendicular to the longitudinal axis of the container and located halfway between the uppermost point and the highest point, exhibits less than 180° circumferential contact with the inner wall, typically less than 120°. (Circumferential contact indicates the extent to which the gel contacts the tube inner wall in a plane substantially perpendicular to the longitudinal axis of the tube.) Another way to describe this embodiment is that it is a configuration where, over 140 to 220° of circumferential contact, the gel exhibits a substantially uniform height in the container, relative to the lower end, and where the highest point at which gel contacts the inner wall of the container is about 8 to about 21 mm above the average height of the area having substantially uniform height

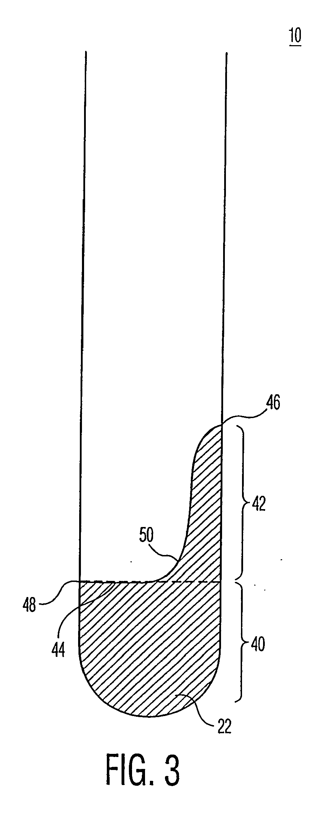

In another embodiment, reflected in Fig. 3, the gel comprises continuous first 40 and second 42 regions, the first region located at or adjacent to the closed lower end of the tube, and the second region extending upward from a portion of the first region.

Typically, the first region comprises an imaginary upper boundary 44 at which the first region exhibits 360° circumferential contact with the inner wall (typically 300 to 360° since some interruptions or regions without gel are possible in this planar upper boundary). The substantially planar upper boundary is typically defined as the surface having a best fit plane within 10° of a plane perpendicular to the longitudinal axis of the tube.

Typically, the uppermost point 46 of the second region is located at least about 8 mm higher than the uppermost point 48 of the upper boundary 44, more typically about 8 to about 21 mm.

Typically, the first region contains at least 80 voL% of the total gel, more typically at least 90 vol.%, with a typical upper limit being 95%.

The interior surface of the gel at the intersection 50 of the first and second regions is generally concave, and typically exhibits a radius of curvature of 4 to 8 mm. (The radius of curvature is defined as the radius of a best-fit sphere along that intersection.)

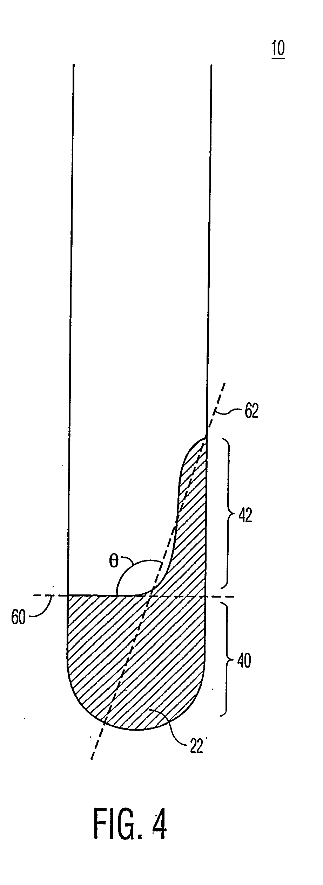

Typically, as reflected in Fig. 4, a best-fit plane 60 to the exposed surface of the first region facing the interior of the container exhibits an angle of 25° or less, more typically 10° or less, with a plane substantially perpendicular to the longitudinal axis of the container. The exposed surface of the second region facing the interior of the container defines a best-fit plane 62 exhibiting a 45 to 90° angle with a plane substantially perpendicular to the longitudinal axis of the container. (Best-fit plane indicates a plane that mathematically best fits the contour of the described surface or outline.)

Typically, the best fit plane to the exposed surface of the first region facing the interior of the container exhibits an angle θ of 90 to 140° with the best-fit plane to the surface of the second region facing the interior of the container.

Typically, along a plane perpendicular to the longitudinal axis of the container located halfway between the average height of the exposed surface of the first region and the uppermost point of the second region, the second region exhibits 80 to 140° circumferential contact with the inner surface.

The entirety of the second region exhibits less than 180° circumferential contact with the inner wall, preferably less than 120°.

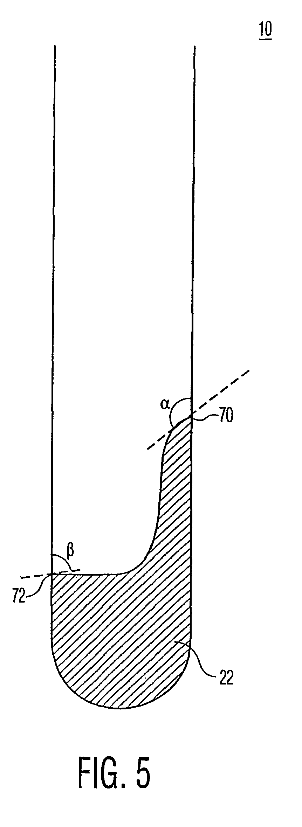

In a further embodiment, reflected in Fig. 5, at the uppermost point at which the gel contacts the inner wall, the angle between the inner wall and the tangent to the gel surface at the point of contact with the inner wall is 100 to 180°, and wherein, at the highest point at which the gel contacts the inner wall opposite the uppermost point, the angle between the inner wall and the tangent to the gel surface at the point of contact with the inner wall is 70 to 100°.

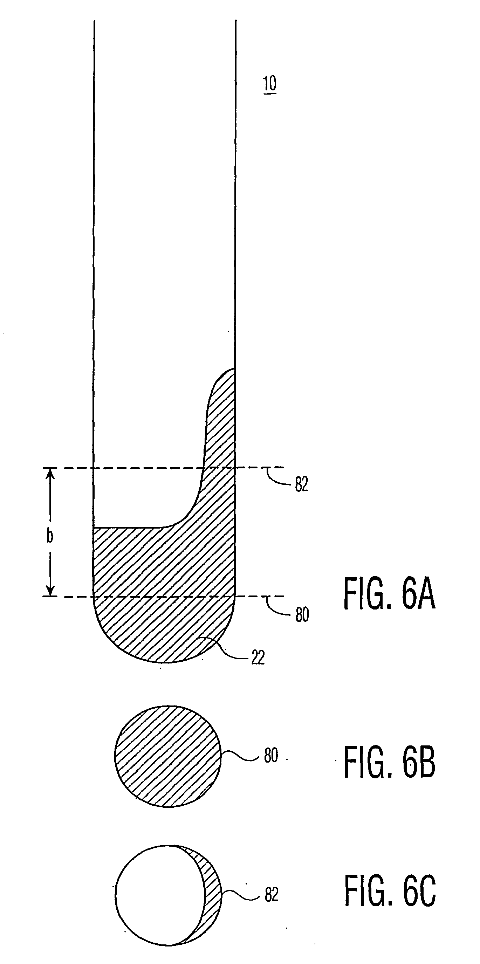

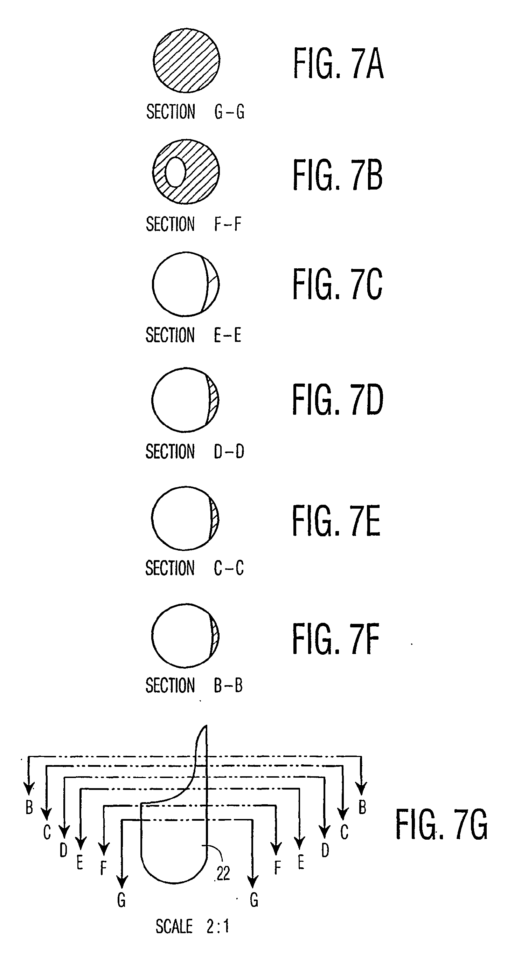

In another embodiment, reflected in Figs. 6A to 6C, upon superimposing on the gel first 80 and second 82 planes perpendicular to the longitudinal axis and spaced a distance b apart, the intersection between the first plane 80 and the gel defines a filled substantially circular or substantially elliptical shape, and the intersection between the gel and the second plane 82 defines a filled substantially crescent or substantially half-moon shape, such as shown in Fig. 6C, with b being a distance less than the distance between the uppermost point of gel contact with the tube inner wall and the bottom of the tube, and greater than the distance between the highest point of gel contact opposite the uppermost point and the bottom of the tube. Typical values for b are greater than 15 mm and less than 26 mm. An example of the cross-section of this embodiment at numerous locations is shown by Figs. 7A to 7G. In particular, Figs. 7A-7F shows the gel geometry at numerous cross-sections of the tube.

In another embodiment, about 5 to about 20 vol.%, optionally 10 to 20 vol.%, of the gel is located within 8 to 12 mm of the uppermost point at which the gel contacts the inner wall.

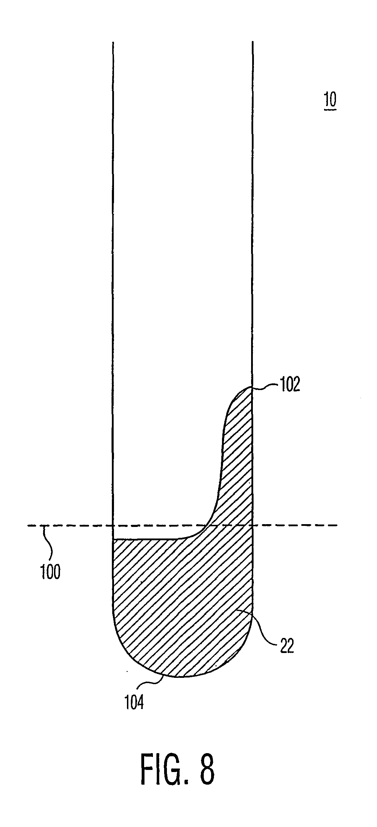

In a further embodiment, reflected in Fig. 8, 10 to 40 vol.%, more typically 20 to 40 vol.% of the gel 22 is located above a plane 100 perpendicular to the longitudinal axis and located halfway between the uppermost point 102 of the gel and the lowermost point 104 of the gel.

Common to all these embodiments is the advantage that they provide, both in gel movement and manufacturability. Gel movement is enhanced by the portion of the gel that extends toward the open end of the tube, e.g., the second region. Specifically, it is believed that providing such a gel extension toward the open end of the tube promotes initiation of gel movement at lower centrifugation speeds than would otherwise be required. The parameters for the gel geometry/placement herein provide for such a region that enhances gel movement upon centrifugation. Moreover, the geometry of the gel is readily attainable in manufacture, as discussed in more detail below.

A variety of separator gels, known in the art, are capable of being advantageously used in the invention. See, e.g., U.S.-A-4,101,422, 4,148,764, and 4,350,593. In particular, acrylic-based, polyester-based, and hydrocarbon-based gels have all been found to be of use as separator materials, where such gels typically contain a resin modified with a particle such as fumed silica in order to form a networked gel.

Both plastic and glass tubes are possible. It is possible to dispose the gel into a tube by a variety of techniques. Generally, a nozzle capable of being inserted into the interior of the tube is used, with either the nozzle, the tubes, or both being moveable for that purpose. Dispensing of gel through the nozzle is normally initiated with the nozzle close to the desired location for the gel (to avoid putting gel on undesired regions of the tube), and as dispensing continues, the nozzle is then slowly drawn up the tube to avoid immersion in the gel. The gel is typically dispensed using pressure or other techniques known in the art. In addition, a tray of tubes is generally processed row by row to expedite manufacturing.

The desired geometry may be provided by various techniques. For example, it is possible to dispose gel into a tube using a nozzle, and then centrifuge the tubes at a particular angle and speed to provide the desired geometry. Such centrifuging may be done with an entire tray of tubes.

It is also possible to dispose the gel into a tube (or group of tubes) while holding the tube at an angle, or by angling the tubes during or after placing the gel into the tubes. The angle and gel deposition steps are controlled to provide the desired geometry. The tubes may then be left at ambient temperature, either at an angle or vertical. Some slumping of the gel may occur, such slumping taken into account when determining the steps necessary to reach the desired geometry.

It is also possible to use a nozzle having an opening oriented at an angle to the tube's axis. For example, the nozzle opening is positioned such that gel is disposed at an angle to the longitudinal axis, i.e., at an angle to vertical (more than one such off-axis nozzle opening is also possible). (There are a variety of techniques to configure a nozzle opening to dispose gel in this manner, including an opening at an angle to the axis of the nozzle, or an angled nozzle tip.) The angled nozzle opening is able to dispense gel in an asymmetric geometry in the tube. Useful angles for such an angled nozzle opening or angle nozzle tip are 25 to 45° to the longitudinal axis of the overall nozzle device, advantageously 45°.

In some cases, it has been found that dispensing the gel under conditions (shear, temperature, viscosity, etc.) that allows the gel to slump from its initial dispensed position, to a final (prior to blood collection and centrifuge) position can be used advantageously to provide a desired geometry such as shown in the Figures. Such slumping may occur under ambient conditions post-dispensing, with the tube remaining in a vertical or angled position, e.g., the tube or tubes are simply moved to a location at which slumping and hardening are allowed to occur - no further actions (e.g., centrifuging) are required to obtain the advantageous geometry. If such slumping is desired, the gel may be dispensed in a manner that provides significant shear, such that the gel exhibits properties that allow such slumping. Conventionally, those in the art would seek to avoid such shear, to prevent such slumping after a dispensing step.

Specific conditions for the gel dispensing depends on, among other things, gel type, tube size, gel dispensing apparatus and techniques, and gel volume, as known to those skilled in the art.

Once the gel is allowed to slump and harden, the tube of the invention generally must go through additional processing steps. For examples, additives useful in blood or urine analysis, e.g., procoagulants or anticoagulants, may be disposed into the tube. As known in the act, blood analysis is often performed on serum, and procoagulants are typically used to enhance the rate of clotting. Such procoagulants include silica particles or enzyme clot activators such as elagic acid, fibrinogen and thrombin. If plasma is desired for analysis, an anticoagulant is generally used to inhibit coagulation, such that blood cells can be separated by centrifugation. Such anticoagulants include chelators such as oxalates, citrate, and EDTA, and enzymes such as heparin. Additives are disposed in the containers in any suitable manner, liquid or solid, including dissolution in a solvent, or disposing in powdered, crystallized, or lyophilized form.

Then, after any such additional additives are put into the tube, the tube (or group of tubes) is subjected to an evacuated chamber with a pressure below atmospheric pressure. A seal such as an elastomeric stopper or pierceable membrane is applied, and the tube is sterilized by a process such as irradiation (e.g., with cobalt 60 radiation), ethylene oxide gas exposure, or electron-beam exposure. (Note that several of these steps may be performed in an order other than that presented above).

The containers of the invention are capable of being formed in any desired size. For example, standard blood collection tubes with outside diameters of 13 x 75 mm or 16 x 100 mm are contemplated.

Other embodiments of the invention will be apparent to those skilled in the art from consideration of the specification and practice of the invention disclosed herein. For example, while the gel geometry of the above embodiments reflects a single region that sweeps upward from a larger region of gel, it is possible to have more than one region sweeping upward, or to have one or more thin regions of gel (e.g., beads of gel) sweep upward.

## Claims

1. A container (10) comprising:
an upper end (12), a lower end (14), and a sidewall (16) between the upper and lower ends (12, 14) having inner and outer walls (18, 20); and a gel (22) located in the container (10) in contact with a portion of the inner wall (18), wherein the gel (22) comprises continuous first and second regions (40, 42), the first region (40) located at or adjacent to the lower end (14), and the second region (42) extending upward from a portion of the first region (40), **characterized in that** the first region (40) comprises an imaginary upper boundary (44) at which the first region (40) exhibits 360° circumferential contact with the inner wall (18) wherein the first region (40) comprises at least 80 vol.% of the gel and wherein the entirety of the second region (42) exhibits less than 180° circumferential contact with the inner wall (18).

2. The container (10) of claim 1, wherein the imaginary upper boundary (44) exhibits a best fit plane within 10° of a plane perpendicular to the longitudinal axis of the tube.

3. The container (10) of claim 1, wherein the uppermost point (46) of the second region (42) is located at least 8 mm higher than the uppermost point (48) of the first region (40).

4. The container (10) of claim 3, wherein the distance is 8 to 21 mm.

5. The container (10) of claim 1, wherein the first region (40) comprises 80 vol.% to 95 vol.% of the gel (22).

6. The container (10) of claim 1, wherein the interior surface of the gel (22) at the intersection of the first and second regions (40, 42) exhibits a radius of curvature between 4 and 8 mm.

7. The container (10) of claim 1, wherein a best-fit plane to the exposed surface of the first region (40) facing the interior of the container (10) exhibits an angle of 25° or less with a plane substantially perpendicular to the longitudinal axis of the container (10).

8. The container (10) of claim 7, wherein the exposed surface of the second region (42) facing the interior of the container (10) defines a best-fit plane exhibiting a 45 to 90° angle with a plane substantially perpendicular to the longitudinal axis of the container (10).

9. The container (10) of claim 1, wherein the best-fit plane to the exposed surface of the first region (40) facing the interior of the container (10) exhibits an angle of 90 to 140° with the best-fit plane to the surface of the second region (42) facing the interior of the container (10).

10. The container (10) of claim 1, wherein the first region (40) comprises an upper boundary (44) at which the first region exhibits 300 to 360° circumferential contact with the inner wall (18).

11. The container (10) of claim 1, wherein, along a plane perpendicular to the longitudinal axis of the container (10) located halfway between the average height of the exposed surface of the first region (40) and the uppermost point (46) of the second region (42), the second region (42) exhibits 80 to 140° circumferential contact with the inner surface.

12. The container (10) of claim 1, wherein the entirety of the second region (42) exhibits less than 120° circumferential contact with the inner wall (18).

13. The container (10) of claim 1, wherein the gel (22) is a thixotropic gel.

14. The container (10) of claim 1, wherein the container (10) is a tube.

15. The container (10) of claim 14, wherein the tube comprises a pierceable closure (24) therein.

16. The container (10) of claim 1, wherein the lower end (14) is closed, and wherein the gel (22) is disposed at the closed lower end (14).

17. A process for fabricating the container (10) of any of claims 1 to 16 comprising the steps of:
providing a container (10) having an open upper end (12), a lower end (14), and a sidewall (16) between the upper and lower ends (12, 14) having inner and outer walls (18, 20);
disposing into the container (10) a gel (22) at an angle to the longitudinal axis of the container (10); and
allowing the gel (22) to slump.

18. The process of claim 17, wherein the gel (22) is disposed through an elongate nozzle having a nozzle opening that directs the gel (22) at the angle to the longitudinal axis of the container (10).

19. The process of claim 18, wherein the angle is less than 90°.

20. The process of claim 17, wherein the container (10) is arranged during the disposing step such that the longitudinal axis of the container (10) is aligned substantially vertically.

21. The process of claim 17, wherein the gel (22) is allowed to slump while the container (10) is arranged such that the longitudinal axis of the container (10) is aligned substantially vertically.

22. The process of claim 17, wherein the gel (22) is disposed at the lower end (14) of the tube.

23. The process of claim 17, wherein the angle is 25 to 40°.

## Patentansprüche

1. Behälter (10), umfassend:
ein oberes Ende (12), ein unteres Ende (14) und eine Seitenwand (16) zwischen dem unteren und dem oberen Ende (12, 14), die eine Innen- und eine Außenwand (18, 20) aufweist; und ein Gel (22), das sich in dem Behälter (10) in Kontakt mit einem Teil der Innenwand (18) befindet, wobei das Gel (22) einen kontinuierlichen ersten und einen kontinuierlichen zweiten Bereich (40, 42) umfasst, wobei sich der erste Bereich (40) am oder in der Nähe des unteren Endes (14) befindet und sich der zweite Bereich (42) ausgehend von einem Teil des ersten Bereichs (40) nach oben erstreckt, **dadurch gekennzeichnet, dass** der erste Bereich (40) eine imaginäre obere Grenze (44) umfasst, an der der erste Bereich (40) einen um 360° umlaufenden Kontakt mit der Innenwand (18) aufweist, wobei der erste Bereich (40) wenigstens 80 Vol.-% des Gels umfasst und wobei die Gesamtheit des zweiten Bereichs (42) weniger als 180° umlaufenden Kontakt mit der Innenwand (18) aufweist.

2. Behälter (10) gemäß Anspruch 1, wobei die imaginäre obere Grenze (44) eine Regressionsebene innerhalb von 10° einer Ebene senkrecht zur Längsachse des Röhrchens aufweist.

3. Behälter (10) gemäß Anspruch 1, wobei sich der oberste Punkt (46) des zweiten Bereichs (42) um wenigstens 8 mm höher befindet als der oberste Punkt (48) des ersten Bereichs (40).

4. Behälter (10) gemäß Anspruch 3, wobei der Abstand 8 bis 21 mm beträgt.

5. Behälter (10) gemäß Anspruch 1, wobei der erste Bereich (40) 80 Vol.-% bis 95 Vol.-% des Gels (22) umfasst.

6. Behälter (10) gemäß Anspruch 1, wobei die Innenfläche des Gels (22) an der Überschneidung des ersten und des zweiten Bereichs (40, 42) einen Krümmungsradius zwischen 4 und 8 mm aufweist.

7. Behälter (10) gemäß Anspruch 1, wobei eine Regressionsebene zur exponierten Oberfläche des ersten Bereichs (40), die zum Innern des Behälters (10) gerichtet ist, einen Winkel von 25° oder weniger mit einer Ebene im Wesentlichen senkrecht zur Längsachse des Behälters (10) aufweist.

8. Behälter (10) gemäß Anspruch 7, wobei die exponierte Oberfläche des zweiten Bereichs (42), die zum Innern des Behälters (10) gerichtet ist, eine Regressionsebene definiert, die einen Winkel von 45 bis 90° mit einer Ebene im Wesentlichen senkrecht zur Längsachse des Behälters (10) aufweist.

9. Behälter (10) gemäß Anspruch 1, wobei die Regressionsebene zur exponierten Oberfläche des ersten Bereichs (40), die zum Innern des Behälters (10) gerichtet ist, einen Winkel von 90 bis 140° mit der Regressionsebene zur Oberfläche des zweiten Bereichs (42), die zum Innern des Behälters (10) gerichtet ist, aufweist.

10. Behälter (10) gemäß Anspruch 1, wobei der erste Bereich (40) eine obere Grenze (44) umfasst, an der der erste Bereich einen um 300 bis 360° umlaufenden Kontakt mit der Innenwand (18) aufweist.

11. Behälter (10) gemäß Anspruch 1, wobei entlang einer Ebene senkrecht zur Längsachse des Behälters (10), die sich in der Mitte zwischen der durchschnittlichen Höhe der exponierten Oberfläche des ersten Bereichs (40) und dem obersten Punkt (46) des zweiten Bereichs (42) befindet, der zweite Bereich (42) einen um 80 bis 140° umlaufenden Kontakt mit der Innenfläche aufweist.

12. Behälter (10) gemäß Anspruch 1, wobei die Gesamtheit des zweiten Bereichs (42) weniger als 120° umlaufenden Kontakt mit der Innenwand (18) aufweist.

13. Behälter (10) gemäß Anspruch 1, wobei das Gel (22) ein thixotropes Gel ist.

14. Behälter (10) gemäß Anspruch 1, wobei der Behälter (10) ein Röhrchen ist.

15. Behälter (10) gemäß Anspruch 14, wobei das Röhrchen einen durchstechbaren Verschluss (24) enthält.

16. Behälter (10) gemäß Anspruch 1, wobei das untere Ende (14) geschlossen ist und wobei sich das Gel (22) am geschlossenen unteren Ende (14) befindet.

17. Verfahren zur Herstellung des Behälters (10) gemäß einem der Ansprüche 1 bis 16, umfassend die Schritte:
Bereitstellen eines Behälters (10), der ein offenes oberes Ende (12), ein unteres Ende (14) und eine Seitenwand (16) zwischen dem unteren und dem oberen Ende (12, 14) mit einer Innen- und einer Außenwand (18, 20) aufweist;
Einbringen eines Gels (22) in den Behälter (10) unter einem Winkel zur Längsachse des Behälters (10); und
Abrutschenlassen des Gels (22).

18. Verfahren gemäß Anspruch 17, wobei das Gel (22) durch eine längliche Düse mit einer Düsenöffnung, die das Gel (22) unter dem Winkel zur Längsachse des Behälters (10) lenkt, eingebracht wird.

19. Verfahren gemäß Anspruch 18, wobei der Winkel kleiner ist als 90°.

20. Verfahren gemäß Anspruch 17, wobei der Behälter (10) während des Schritts des Einbringens so angeordnet ist, dass die Längsachse des Behälters (10) im Wesentlichen senkrecht ausgerichtet ist.

21. Verfahren gemäß Anspruch 17, wobei das Gel (22) abrutschen gelassen wird, während der Behälter (10) so angeordnet ist, dass die Längsachse des Behälters (10) im Wesentlichen senkrecht ausgerichtet ist.

22. Verfahren gemäß Anspruch 17, wobei das Gel (22) am unteren Ende (14) des Röhrchens eingebracht wird.

23. Verfahren gemäß Anspruch 17, wobei der Winkel 25 bis 40° beträgt.

## Revendications

1. Récipient (10), comprenant :
une extrémité supérieure (12), une extrémité inférieure (14) et une paroi latérale (16) entre lesdits extrémités supérieure et inférieure (12, 14) ayant des parois intérieure et extérieure (18, 20) ; et un gel (22) situé dans le récipient (10) en contact avec une partie de la paroi intérieure (18), dans lequel le gel (22) comprend une première et une seconde régions continues (40, 42), la première région (40) se trouvant au niveau ou à proximité adjacente de l'extrémité inférieure (14), et la seconde région (42) s'étendant en haut à partir d'une parte de la première région (40), **caractérisé en ce que** ladite première région (40) comprend une limite supérieure imaginaire (44) au niveau de laquelle la première région (40) présente un contact de 360° de la circonférence avec la paroi intérieure (18), dans lequel la première région (40) comprend au moins 80 % en volume du gel et dans lequel la totalité de la seconde région (42) présente un contact de moins de 180° de la circonférence avec la paroi intérieure (18).

2. Récipient (10) selon la revendication 1, dans lequel ladite limite supérieure imaginaire (44) présente un plan de régression à 10° près dans un plan perpendiculaire à l'axe longitudinal du tube.

3. Récipient (10) selon la revendication 1, dans lequel le point le plus élevé (46) de la seconde région (42) se trouve au moins 8 mm plus haut que le point le plus élevé (48) de la première région (40).

4. Récipient (10) selon la revendication 3, dans lequel la distance est de 8 à 21 mm.

5. Récipient (10) selon la revendication 1, dans lequel la première région (40) comprend 80 % en volume à 95 % en volume du gel (22).

6. Récipient (10) selon la revendication 1, dans lequel la surface intérieure du gel (22) à l'intersection des première et seconde régions (40, 42) présente un rayon de courbure compris entre 4 et 8 mm.

7. Récipient (10) selon la revendication 1, dans lequel un plan de régression ajusté à la surface exposée de la première région (40) tournée vers l'intérieur du récipient (10) présente un angle de 25° ou moins avec un plan sensiblement perpendiculaire à l'axe longitudinal du récipient (10).

8. Récipient (10) selon la revendication 7, dans lequel la surface exposée de la seconde région (42) tournée vers l'intérieur du récipient (10) définit un plan de régression présentant un angle de 45 à 90° avec un plan sensiblement perpendiculaire à l'axe longitudinal du récipient (10).

9. Récipient (10) selon la revendication 1, dans lequel le plan de régression ajusté à la surface exposée de la première région (40) tournée vers l'intérieur du récipient (10) présente un angle de 90 à 140° avec le plan de régression ajusté à la surface de la seconde région (42) tournée vers l'intérieur du récipient (10).

10. Récipient (10) selon la revendication 1, dans lequel la première région (40) comprend une limite supérieure (44) au niveau de laquelle la première région présente un contact de 300 à 360° de la circonférence avec la paroi intérieure (18).

11. Récipient (10) selon la revendication 1, dans lequel, le long d'un plan perpendiculaire à l'axe longitudinal du récipient (10) situé à mi-chemin entre le niveau moyen de la surface exposée de la première région (40) et le point le plus élevé (46) de la seconde région (42), la seconde région (42) présente un contact de 80 à 140° de la circonférence avec la surface intérieure.

12. Récipient (10) selon la revendication 1, dans lequel la totalité de la seconde région (42) présente un contact de moins de 120° de la circonférence avec la paroi intérieure (18).

13. Récipient (10) selon la revendication 1, dans lequel le gel (22) est un gel thixotropique.

14. Récipient (10) selon la revendication 1, dans lequel le récipient (10) est un tube.

15. Récipient (10) selon la revendication 14, dans lequel ledit tube comprend une fermeture perçable (24) dedans.

16. Récipient (10) selon la revendication 1, dans lequel l'extrémité inférieure (14) est fermée et dans lequel le gel (22) est situé au niveau de l'extrémité inférieure (14) fermée.

17. Procédé pour fabriquer le récipient (10) selon l'une quelconque des revendications 1 à 16, comprenant les étapes consistant à :
procurer un récipient (10) ayant une extrémité supérieure (12) ouverte, une extrémité inférieure (14) et une paroi latérale (16) entre lesdites extrémités supérieure et inférieure (12, 14) ayant des parois intérieure et extérieure (18, 20) ;
placer dans le récipient (10) un gel (22) à un angle avec l'axe longitudinal du récipient (10) ; et
laisser glisser le gel (22).

18. Procédé selon la revendication 17, dans lequel le gel (22) est placé à travers une buse allongée ayant un orifice de buse qui dirige le gel (22) à l'angle avec l'axe longitudinal du récipient (10).

19. Procédé selon la revendication 18, dans lequel ledit angle est moins de 90°.

20. Procédé selon la revendication 17, dans lequel le récipient (10), pendant l'étape de placer, est disposé de telle manière que l'axe longitudinal du récipient (10) soit aligné sensiblement verticalement.

21. Procédé selon la revendication 17, dans lequel le gel (22) est laissé glisser pendant que le récipient (10) est arrangé de telle manière que l'axe longitudinal du récipient (10) soit aligné sensiblement verticalement.

22. Procédé selon la revendication 17, dans lequel le gel (22) est placé à l'extrémité inférieure (14) du tube.

23. Procédé selon la revendication 17, dans lequel ledit angle est de 25 à 40°.
